# EUROPEAN PATENT SPECIFICATION

(11) **EP 3 345 559 B1**
(45) Date of publication and mention of the grant of the patent: **14.10.2020**
(21) Application number: 16841589.1
(22) Date of filing: 23.08.2016
(51) Int. Cl.: A61B 17/43, A61B 17/435, A61K 49/22, A61L 29/18

(54) **INSTRUMENT FOR EMBRYO TRANSPLANTATION AND DEVICE FOR EMBRYO TRANSPLANTATION**
INSTRUMENT ZUR EMBRYOTRANSPLANTATION UND VORRICHTUNG ZUR EMBRYOTRANSPLANTATION
INSTRUMENT DE TRANSPLANTATION D'EMBRYONS ET DISPOSITIF DE TRANSPLANTATION D'EMBRYONS

(30) Priority: 01.09.2015 JP 2015171915
(43) Date of publication of application: 11.07.2018
(73) Proprietor: Kitazato Corporation, Fuji-shi Shizuoka 4160907 (JP)
(72) Inventor: INOUE Futoshi, Fuji-shi Shizuoka 4160907 (JP)
(74) Representative: Casalonga
(86) International application number: PCT/JP2016/074501
(87) International publication number: WO 2017/038557

(56) References cited:
- EP-A1- 0 624 342
- EP-A1- 2 080 481
- EP-A2- 2 620 111
- JP-A- H1 119 094
- JP-A- 2004 298 632
- JP-A- 2007 236 451
- JP-A- 2007 523 716
- US-A- 5 201 314
- US-A1- 2003 032 896
- US-A1- 2003 040 756

## Description

### TECHNICAL FIELD

The present invention relates to an embryo transfer tool and an embryo transfer device. More specifically, the present invention relates to an embryo transfer tool and an embryo transfer device to be used in transferring an embryo (fertilized ovum) fertilized in vitro to the uterus of a living body, namely, to the uterus of an animal.

In particular, the present invention relates to an embryo transfer tool according to the preamble of claim 1, such as it is for example known from US2003/040756A1, and an embryo transfer device comprising such an embryo transfer tool.

### BACKGROUND ART

In transferring an embryo into the uterus, it is general to collect ova and sperms, put the ova in a culture vessel, add a sperm suspension to the ova, namely, fertilize the ova with sperms, and transfer a fertilized ovum or an embryo obtained by dividing the fertilized ovum into two parts, four parts or eight parts to the uterus of an animal, for example, a human uterus.

Thereafter a comparatively hard sheath is inserted into the uterus from the vaginal opening and passed through the cervical canal. Thereafter the embryo is directly inserted into the sheath or a tube which has sucked the embryo thereto is inserted thereinto. Thereafter the tube is pressed into the sheath. After the front end portion of the tube reaches the cervical opening, a syringe is pressed to transfer the embryo into the uterus. Thereafter the tube and a mantle tube are removed from the sheath. In this manner, the transfer of the embryo finishes.

The present inventors proposed an embryo transfer device as disclosed in Japanese Patent Application Laid-Open Publication No. 2004-129789 (patent document 1). The embryo transfer device 1 of the patent document 1 has the flexible sheath 2 having the path penetrating therethrough from its front end to its rear end and the spherical bulged part 22 provided on the outer surface of the front end thereof, the flexible stylet 3 which is removably inserted into the flexible sheath and whose front end projects a little from the front end surface of the sheath, and the transfer tube body 4 having he flexible front end part 41a which can be inserted into the flexible sheath 2 from which the stylet has been removed and can be projected in a predetermined length from the front end of the flexible sheath 2.

The transfer tube body 4 to be used for the embryo transfer device is desired to have a high ultrasound imaging property at its front end portion. The transfer tube bodies having a high ultrasound imaging property are proposed, as disclosed in Japanese Patent Application Laid-Open Publication No. 2003-190275 (patent document 2) and Japanese Patent Application Laid-Open Publication No. 2004-298632 (patent document 3).

In the surgical medical device (1, 1') made of a plastic material disclosed in the patent document 2, the plastic material contains gas bubbles (12, 12') in the major part of the thickness thereof at at least one selected portion of the device to allow the device to have visibility in an ultrasound imaging operation. The embryo replacing catheter having the flexible shaft 1 formed by press molding transparent polyurethane is also disclosed in the patent document 2. The shaft 1 has the hole 10 extended longitudinally. The gas bubbles 12 whose diameters are in the range of 5 µm to 10µm are introduced into the wall of the shaft in the thickness direction thereof by adding a gas to the transparent polyurethane while it is being press molded. In the disclosure, the number of the bubbles 12 is so selected as to increase the visibility of the catheter while the ultrasound imaging operation is being performed and view a substance flowing along the catheter.

The catheter for transporting an embryo or another medical device disclosed in the patent document 3 has the shaft 1 having two layers 12 and 13 formed by press molding. The outer layer 13 is comparatively thick and contains the bubbles 22 whose number is large enough to improve the visibility of the catheter in ultrasound observation. The density of the bubbles is so set that the substance inside the catheter can be viewed with the naked eye. The inner layer 12 is comparatively thin and does not contain bubbles in order to allow the catheter to have the smooth hole 10.

### PRIOR ART DOCUMENTS

### PATENT DOCUMENTS

Patent document 1: Japanese Patent Application Laid-Open Publication No. 2004-129789
Patent document 2: Japanese Patent Application Laid-Open Publication No. 2003-190275
Patent document 3: Japanese Patent Application Laid-Open Publication No. 2004-298632

### SUMMARY OF THE INVENTION

### PROBLEMS TO BE SOLVED BY THE INVENTION

Each of the transfer tube bodies of the patent documents 2 and 3 has a sufficiently high ultrasound imaging property. Each of the transfer tube bodies contains a large number of bubbles, which causes a portion of the tube to become brittle. An embryo accommodated in the transfer tube is transferred to a person to which the embryo is to be transferred. Thus, both tube bodies are preferable in that by microscopic observation, an operator can check whether the embryo has been securely accommodated in the transfer tube. In the disclosure of the documents 2 and 3, the transfer tube may be partly provided with the bubble-containing part. But in dependence on a method of forming the bubble-containing part, there is a case in which the ultrasound imaging property becomes insufficient and conversely, there is a case in which the tube has a low degree of visibility because the bubble-containing part contains a large number of bubbles when the inside thereof is microscopically observed.

It is an object of the present invention to provide an embryo transfer tool which has a sufficiently high ultrasound imaging property, has sufficiently high optical transparency and visibility in microscopically observing the inside thereof by using a light source, and does not have a brittle portion partially formed in a tube wall thereof and provide an embryo transfer device using the embryo transfer tool.

### MEANS FOR SOLVING THE PROBLEMS

The above-described object can be achieved by the following tool.

An embryo transfer tool comprising a soft tube having a lumen penetrating therethrough from a distal end thereof to a proximal end thereof and a hub provided at a proximal end portion of said soft tube,
wherein said soft tube has a first bubble-containing surface layer extended in a predetermined width and in a predetermined length from said distal end of said soft tube toward said proximal end thereof, a second bubble-containing surface layer extended in a predetermined width from said distal end of said soft tube toward said proximal end thereof and opposed to said first bubble-containing surface layer, a first colorless and transparent part positioned between said first and second bubble-containing surface layers, extended in a predetermined length from said distal end of said soft tube toward said proximal end thereof, and allowing an inside of said lumen to be visually recognized by microscopic observation, and a second colorless and transparent part provided opposite to said first colorless and transparent part; and
each of said first and second bubble-containing surface layers has a lot of bubbles set long in an axial direction of said soft tube; a thickness of each of said first and second bubble-containing surface layers is set to 1/5 to 1/3 of a thickness of said soft tube; a width of each of said first and second bubble-containing surface layers is set to 5/100 to 20/100 of an outer circumferential length of said soft tube; and a width of each of said first and second colorless and transparent parts is set to not less than 30/100 of said outer circumferential length of said soft tube.

The above-described object can be achieved by the following device.

An embryo transfer device comprising the above embryo transfer tool and a sheath having a flexible tube harder than said soft tube and a sheath hub provided at a proximal end of said flexible tube, wherein said sheath accommodates said soft tube with a distal part of said soft tube of said embryo transfer tool projecting from a distal end of said sheath.

The thickness of each of the first and second bubble-containing surface layers is set to 1/5 to 1/3 of the thickness of the soft tube. In addition, the bubbles contained in the first and second bubble-containing surface layers are set long in the axial direction of the soft tube. Therefore, the containing of the bubbles hardly causes the portion (bubble-containing layer holding portion) of the soft tube to become brittle. In addition, because the soft tube has the two bubble-containing surface layers opposed to each other, the soft tube has a sufficiently high ultrasound imaging property. Further, because the soft tube has the two colorless and transparent parts each having the width not less than 30/100 of the outer circumferential length of the soft tube, the soft tube has sufficiently high optical transparency and visibility to be obtained by microscopic observation.

### BRIEF DESCRIPTION OF THE DRAWINGS

Fig. 1 is a front view of one embodiment of an embryo transfer tool of the present invention.
Fig. 2 is a front view of an embryo transfer device using the embryo transfer tool shown in Fig. 1.
Fig. 3 is a front view of a sheath used for the embryo transfer device shown in Fig. 2.
Fig. 4 is an enlarged view of a distal end portion of the embryo transfer device shown in Fig. 2.
Fig. 5 is an enlarged view of a distal end portion of the embryo transfer tool shown in Fig. 1.
Fig. 6 is a sectional view taken along a line A-A of Fig. 5.
Fig. 7 is an enlarged sectional view of a proximal end portion of the embryo transfer device shown in Fig. 2.

### MODE FOR CARRYING OUT THE INVENTION

The embryo transfer tool of the present invention and the embryo transfer device of the present invention using the embryo transfer tool are described below by using embodiments shown in the drawings.

An embryo transfer tool 1 of the present invention has a soft tube 11 having a lumen 16 penetrating therethrough from its distal end to its proximal end and a hub 12 provided at a proximal end portion of the soft tube 11. The soft tube 11 has a first bubble-containing surface layer 13a extended in a predetermined width and in a predetermined length from the distal end of the soft tube toward the proximal end thereof, a second bubble-containing surface layer 13b extended in a predetermined width from the distal end of the soft tube toward the proximal end thereof and opposed to the first bubble-containing surface layer 13a, a first colorless and transparent part 15a positioned between the first and second bubble-containing surface layers 13a and 13b, extended in a predetermined length from the distal end of the soft tube 11 toward the proximal end thereof, and allowing the inside of the lumen to be visually recognized by microscopic observation, and a second colorless and transparent part 15b provided opposite to the first colorless and transparent part 15a. Each of the first and second bubble-containing surface layers 13a and 13b has a lot of bubbles 14 set long in an axial direction of the soft tube 11. The thickness of each of the first and second bubble-containing surface layers 13a and 13b is set to 1/5 to 1/3 of the thickness of the soft tube 11. The width of each of the first and second bubble-containing surface layers 13a and 13b is set to 5/100 to 20/100 of the outer circumferential length of the soft tube 11. The width of each of the first and second colorless and transparent parts 15a and 15b is set to not less than 30/100 of the outer circumferential length of the soft tube 11.

The thickness of each of the first and second bubble-containing surface layers 13a and 13b is set to 1/5 to 1/3 of the thickness of the soft tube 11. In addition, the bubbles 14 contained in the first and second bubble-containing surface layers 13a and 13b are set long in the axial direction of the soft tube 11. Therefore, the containing of the bubbles hardly causes the portion (bubble-containing layer holding portion) of the soft tube 11 to become brittle. In addition, because the soft tube has the two bubble-containing surface layers opposed to each other, the soft tube has a sufficiently high ultrasound imaging property. Further, because the soft tube has the two colorless and transparent parts each having the width not less than 30/100 of the outer circumferential length of the soft tube 11, the soft tube has sufficiently high optical transparency and visibility to be obtained by the microscopic observation.

The embryo transfer tool 1 of this embodiment has the soft tube 11 having the lumen penetrating therethrough from its distal end to its proximal end and the hub 12 fixed to the proximal end portion of the soft tube 11. The hub 12 may be formed integrally with the soft tube.

The soft tube 11 serves as a means for transferring an embryo and has the lumen 16 penetrating therethrough from its distal end to its proximal end. The length of the soft tube is set to 70 to 800mm and favorably 200 to 600mm. The outer diameter of the soft tube is set to 0.5 to 3mm and favorably 1 to 2mm. The inner diameter of the soft tube is set to 0.3 to 0.7mm and favorably 0.4 to 0.6mm.

As materials for forming the soft tube 11, those having flexibility to a certain extent are preferable. It is possible to use synthetic rubber such as urethane rubber, silicone rubber, butadiene rubber, natural rubber such as Latex rubber, soft vinyl chloride, polyolefin (polyethylene, polypropylene, ethylene-propylene copolymer, ethylene-vinyl acetate copolymer, mixture of polypropylene and polyethylene or mixture of polypropylene and polybutene), polyester (polyethylene terephthalate, polybutylene terephthalate), polyamide, elastomers such as polyolefin-based elastomer, polyamide-based elastomer, styrene-based elastomer (for example, styrene-butadiene-styrene copolymer, styrene-isoprene-styrene copolymer, styrene-ethylene-butylene-styrene copolymer), polyurethane, and preferably, thermoplastic polyurethane (thermoplastic polyether polyurethane and thermoplastic polyester polyurethane are preferable. Segmented thermoplastic polyether polyurethane having a soft segment portion and a hard segment portion are especially preferable. More specifically, as a main component of the soft segment, polytetramethylene ether glycol, polyethylene glycol, and polypropylene glycol are preferable. As a main component of the hard segment, 1,4-butanediol is preferable). Rubber such as silicone rubber and elastomer are preferable. The silicone rubber is especially preferable. The silicone rubber having 30-90kg/cm² and preferably 40-60kg/ cm² at 200% modulus is used.

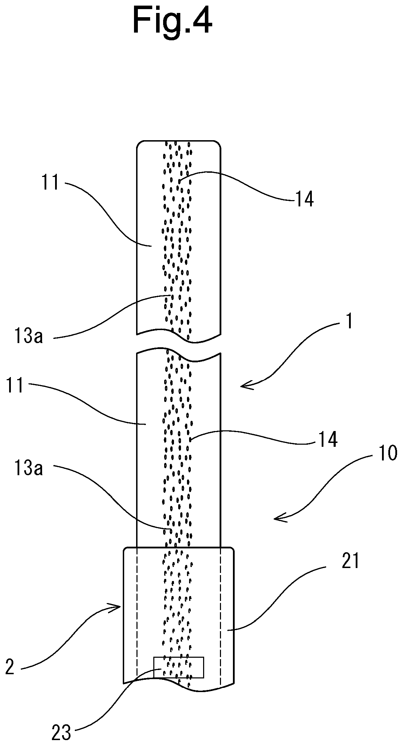

As shown in Figs. 4 through 6, the soft tube 11 has the first bubble-containing surface layer 13a extended in the predetermined width and in the predetermined length from the distal end of the soft tube toward the proximal end thereof and the second bubble-containing surface layer 13b extended in the predetermined width from the distal end of the soft tube toward the proximal end thereof and opposed to the first bubble-containing surface layer 13a through the intermediary of the center of the soft tube 11. In the embryo transfer tool 1 of this embodiment, the first and second bubble-containing surface layers 13a and 13b are extended from the distal end of the soft tube 11 to the proximal end thereof. As shown in Fig. 4, it is necessary for the soft tube to have the first and second bubble-containing surface layers 13a and 13b at a part of a flexible tube 21 of a sheath 2 projected from a distal end of the flexible tube. The first and second bubble-containing surface layers may not necessarily be formed at a part of the flexible tube disposed proximally from the distal end of the flexible tube.

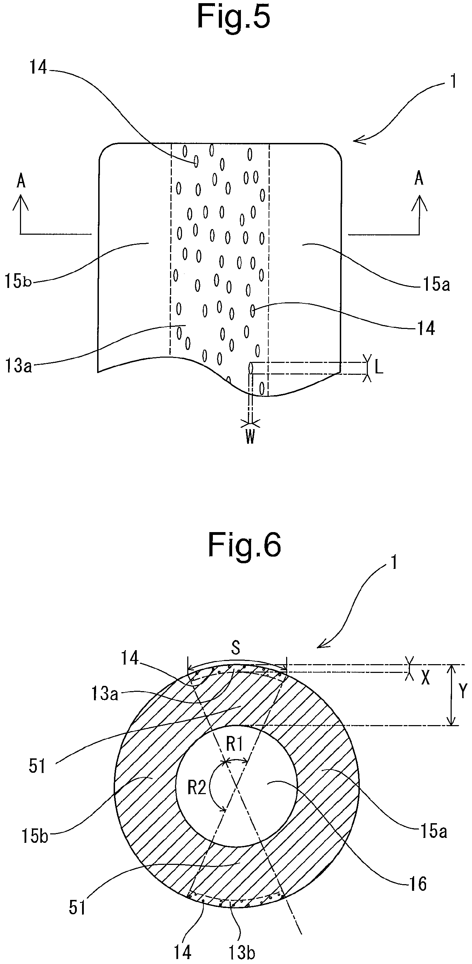

As shown in Figs. 5 and 6, each of the first and second bubble-containing surface layers 13a and 13b has a large number of bubbles 14 therein. As shown in Fig. 5, the bubbles 14 are set long in the axial direction of the soft tube 11. As shown in Fig. 5, in the embryo transfer tool 1 of this embodiment, a length L of the bubble 14 in the axial direction of the soft tube 11 is set to not less than twice as long as a width W of the bubble 14. The length L of the bubble 14 in the axial direction of the soft tube 11 is set to favorably 1.5 to 4 times as long as the width W of the bubble 14 and more favorably two to three times as long as the width W thereof. The cross-sectional area of the bubble 14 gradually decreases toward its distal end and its proximal end.

The length L of the bubble 14 in the axial direction of the soft tube is set to favorably 0.01 to 0.80mm and more favorably 0.02 to 0.6mm. The width W of the bubble 14 is set to favorably 0.005 to 0.5mm and more favorably 0.01 to 0.4mm. The bubble volume content of each of the first and second bubble-containing surface layers 13a and 13b is set to favorably 3 to 15 percent by volume and more favorably 5 to 10 percent by volume. The cross-sectional area of the bubble in the axial direction of the soft tube is set to favorably 0.0001 to 0.10mm², more favorably 0.001 to 0.80mm², and most favorably 0.01 to 0.3mm².

As shown in Fig. 6, a thickness X of each of the first and second bubble-containing surface layers 13a and 13b is set to 1/5 to 1/3 of a thickness Y of the soft tube 11. The thickness X is set to favorably 1/5 to 1/4 of the thickness Y of the soft tube 11. A region of the soft tube inward from the first bubble-containing surface layers 13a and that of the soft tube inward from the second bubble-containing surface layer 13b are formed as a layer not containing bubbles. By setting the thickness of each of the first and second bubble-containing surface layers 13a and 13b to the above-described range, the soft tube 11 has the sufficiently thick layer not containing bubbles. Thereby the soft tube becomes hardly brittle at the bubble-containing layer holding portion. The embryo transfer tool 1 of the present invention has a layer 51, not containing bubbles, which has a thickness not less than 2/3 of the thickness Y of the soft tube 11. The layer 51 not containing bubbles is located under each of the first and second bubble-containing surface layers 13a and 13b. It is preferable to set the thickness of the layer 51 not containing bubbles to not less than 3/4 of the thickness Y of the soft tube.

As shown in Figs. 4 through 6, the first and second bubble-containing surface layers 13a and 13b are extended from the distal end of the soft tube 11 toward the proximal end thereof in the predetermined width and in parallel with the central axis of the soft tube 11. In the embryo transfer tool of this embodiment, the first and second bubble-containing surface layers 13a and 13b are extended toward the proximal end of the soft tube in a substantially constant width. As shown in Fig. 6, a width S of each of the first and second bubble-containing surface layers 13a and 13b is set to 5/100 to 20/100 of the outer circumferential length of the soft tube 11. By setting the width of each of the first and second bubble-containing surface layers 13a and 13b to the above-described range, the first and second colorless and transparent parts 15a and 15b are allowed to have a sufficiently large width. It is preferable to set the width S of each of the first and second bubble-containing surface layers 13a and 13b to 7/100 to 15/100 of the outer circumferential length of the soft tube 11. An angle R1 of a part where the first bubble-containing surface layer 13a is formed and that of a part where the second bubble-containing surface layer 13b is formed are set to favorably 15 to 70 degrees and more favorably 30 to 50 degrees. Although it is preferable to set the width of the first bubble-containing surface layer 13a and that of the second bubble-containing surface layer 13b substantially equally to each other, it is possible to differentiate the widths thereof from each other.

As shown in Fig. 6, in the embryo transfer tool 1 of this embodiment, the bubble content of an outer portion of each of the first and second bubble-containing surface layers is set higher than that of an inner portion thereof. That is, the number of bubbles present in the outer portion of each of the bubble-containing surface layers is larger than the number of bubbles present in the inner portion thereof. In the embryo transfer tool 1 of this embodiment, the number of the bubbles contained in each of the bubble-containing layers increases gradually toward an outer surface thereof. In other words, the number of the bubbles contained in each of the bubble-containing layers decreases gradually toward an inner surface thereof. By increasing the number of the bubbles toward the outer surface of each of the bubble-containing layers, it is possible to allow the embryo transfer tool to have an excellent ultrasound imaging property.

As shown in Fig. 6, in the embryo transfer tool 1 of this embodiment, bubble-caused irregularities are not formed on the outer surface of each of the first and second bubble-containing surface layers 13a and 13b, but the outer surface of each of the bubble-containing surface layers is as smooth as the outer surface of a bubble-non-containing part.

The embryo transfer tool 1 has the first colorless and transparent part 15a positioned between the first and second bubble-containing surface layers 13a and 13b and extended in the predetermined length from the distal end of the soft tube 11 toward the proximal end thereof and the second colorless and transparent part 15b provided opposite to the first colorless and transparent part 15a through the intermediary of the central axis of the soft tube. The first and second colorless and transparent parts 15a and 15b are provided opposite to each other and have sufficiently high optical transparency and microscopic visibility inside the lumen 16. Thereby it is possible to check the presence of living cells held inside the lumen by a microscope.

The width of each of the colorless and transparent parts 15a and 15b is set to not less than 30/100 of the outer circumferential length of the soft tube 11. It is preferable to set the width of each of the colorless and transparent parts 15a and 15b to not less than 35/100 of the outer circumferential length of the soft tube 11. It is favorable to set an angle R2 of a region in which the first colorless and transparent part 15a is formed and that of a region in which the second colorless and transparent part 15b is formed to 110 to 165 degrees and more favorable to set the angle R2 to 130 to 150 degrees. Although it is preferable to set the widths of the first and second colorless and transparent parts 15a and 15b substantially equally to each other, the widths thereof may be differentiated from each other.

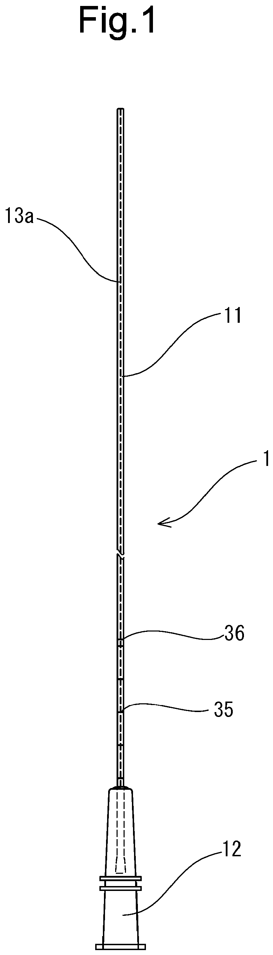

As shown in Fig. 1, the embryo transfer tool 1 of this embodiment has a plurality of markers 35 arranged at regular intervals at the proximal end portion, of the soft tube 11, which is located distally from the hub 12. More specifically, the embryo transfer tool 1 has five distance index markers 35 arranged at equal intervals and an end marker 36 positioned proximately to one distance index marker 35 located most distally and distally therefrom.

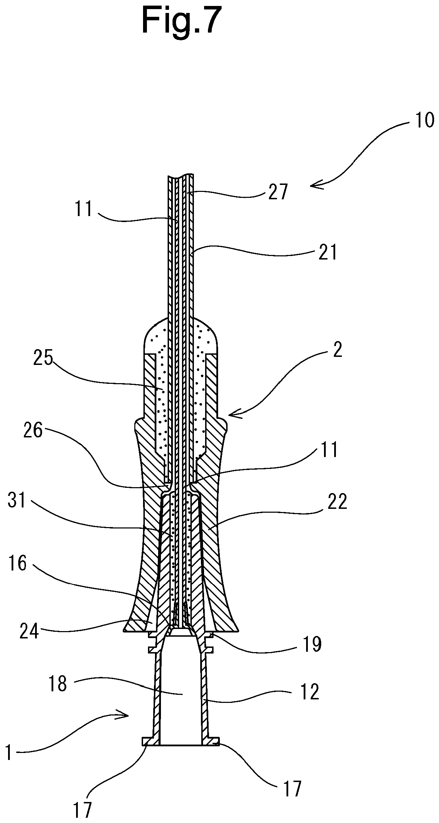

The embryo transfer tool 1 has the hub 12 fixed to the proximal end portion of the soft tube 11. As shown in Fig. 7, the soft tube 11 is fixed to the hub 12 with a caulking member 16 inserted into the proximal end of the soft tube and an adhesive agent 31.

The hub 12 has an open part and a hollow part 18 communicating with the inside of the soft tube 11 at its proximal end. The hollow part 18 is formed as a luer tapered part which can be liquid-tightly mounted on a nozzle of a medical appliance such as a syringe. The hub 12 has two opposed projected parts 17 projected outward from the proximal end thereof. The hub 12 has two annular ribs 19 provided at its central portion. A part of the hub positioned forward from the rib 19 is formed as a tubular part smaller in its diameter than a part thereof positioned rearward from the rib 19. The small-diameter tubular part of the hub can be inserted into a sheath hub 22 of the sheath 2 to be described later. Hard resin is used as the material for forming the hub.

An embryo transfer device 10 of the present invention is described below.

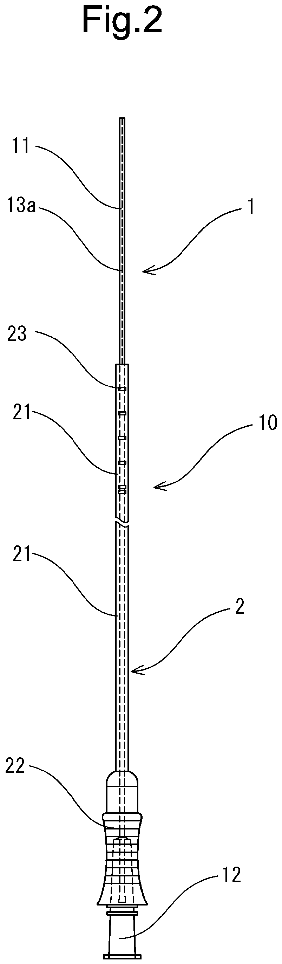

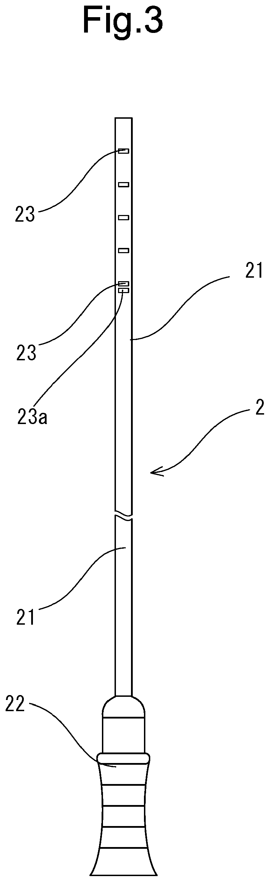

The embryo transfer device 10 is composed of the embryo transfer tool 1 and the sheath 2. As shown in Figs. 2 and 3, the sheath 2 accommodates the soft tube 11 with the distal part of the soft tube 11 of the embryo transfer tool 1 projecting from the distal end of the sheath. The sheath has the flexible tube 21 harder than the soft tube 11 and the sheath hub 22 provided at the proximal end of the flexible tube 21.

The sheath 2 of this embodiment has the flexible tube 21 having a lumen 27 penetrating therethrough from its distal end to its proximal end and the sheath hub 22 fixed to the proximal end portion of the flexible tube 21. The sheath hub 22 may be formed integrally with the flexible tube 21.

As shown in Figs. 2, 3, and 7, the sheath 2 has the flexible tube 21 having the lumen 27 penetrating therethrough from its distal end to its proximal end. The length of the flexible tube 21 is set to 50 to 300mm and preferably 100 to 250mm. The outer diameter of the flexible tube is set to 1 to 5mm and preferably 1.5 to 3.5mm. The inner diameter of the flexible tube is set to 0.8 to 4.8mm and preferably 1.3 to 3.3mm. The length of the part of the soft tube 11 of the embryo transfer tool 1 projected from the distal end of the sheath 2 is set to favorably 30 to 100mm and more favorably 35 to 70mm.

As materials for forming the flexible tube 21, those harder than the material for forming the soft tube 11 and having a certain extent of shape-retaining property and flexibility are used. As the materials for forming the flexible tube 21, it is possible to use polyester, polyolefin (for example, polyethylene, polypropylene, ethylene-propylene copolymer), polyamide (for example, nylon 6, nylon 66), polyester (for example, polyethylene terephthalate), and fluororesin (for example, PTFE, ETFE). As shown in Fig. 3, the sheath 2 of this embodiment has a plurality of insertion depth checking markers 23 formed on the outer surface of the distal part thereof. More specifically, the sheath has five distance index markers 23 arranged at regular intervals and an end marker 23a positioned proximately to one distance index marker 23 located most proximally and proximally therefrom.

As shown in Fig. 7, the sheath hub 22 is fixed to a rear end of the flexible tube 21 with an adhesive agent 25. The sheath hub 22 is a hollow hub having a lumen 24 communicating with the lumen 27 formed inside the flexible tube 21. As shown in Figs. 3 and 7, the sheath hub has a gripping concave part on a side surface thereof and a non-slip rib formed on the surface of the concave part. The sheath hub 22 has an annular rib 26 projected inward inside the lumen 27. The proximal end of the flexible tube 21 is in contact with the annular rib 26. The flexible tube 21 is fixed to the sheath hub 22 with adhesive agent 25 filled in a space between the sheath hub 22 and the proximal end portion of the flexible tube 21. The diameter of an open portion of the sheath hub 22 increases in a tapered manner. Hard resin is used as a material for forming the sheath hub.

### INDUSTRIAL APPLICABILITY

The embryo transfer tool of the present invention has the following construction:
(1) An embryo transfer tool comprising a soft tube having a lumen penetrating therethrough from a distal end thereof to a proximal end thereof and a hub provided at a proximal end portion of said soft tube,
   wherein said soft tube has a first bubble-containing surface layer extended in a predetermined width and in a predetermined length from said distal end of said soft tube toward said proximal end thereof, a second bubble-containing surface layer extended in a predetermined width from said distal end of said soft tube toward said proximal end thereof and opposed to said first bubble-containing surface layer, a first colorless and transparent part positioned between said first and second bubble-containing surface layers, extended in a predetermined length from said distal end of said soft tube toward said proximal end thereof, and allowing an inside of said lumen to be visually recognized by microscopic observation, and a second colorless and transparent part provided opposite to said first colorless and transparent part; and
   each of said first and second bubble-containing surface layers has a lot of bubbles set long in an axial direction of said soft tube; a thickness of each of said first and second bubble-containing surface layers is set to 1/5 to 1/3 of a thickness of said soft tube; a width of each of said first and second bubble-containing surface layers is set to 5/100 to 20/100 of an outer circumferential length of said soft tube; and a width of each of said first and second colorless and transparent parts is set to not less than 30/100 of said outer circumferential length of said soft tube.

The thickness of each of the first and second bubble-containing surface layers is set to 1/5 to 1/3 of the thickness of the soft tube. In addition, the bubbles contained in the first and second bubble-containing surface layers are set long in the axial direction of the soft tube. Therefore, the containing of the bubbles hardly causes the portion (bubble-containing layer holding portion) of the soft tube to become brittle. In addition, because the soft tube has the two bubble-containing surface layers opposed to each other, the soft tube has a sufficiently high ultrasound imaging property. Further, because the soft tube has the two colorless and transparent parts each having the width not less than 30/100 of the outer circumferential length of the soft tube, the soft tube has sufficiently high optical transparency and visibility to be obtained by microscopic observation.

## Claims

1. An embryo transfer tool (1) comprising a soft tube (11) having a lumen penetrating therethrough from a distal end thereof to a proximal end thereof and a hub (12, 22) provided at a proximal end portion of said soft tube (11),
wherein said soft tube (11) has a first bubble-containing surface layer (13a) extended in a predetermined width and in a predetermined length from said distal end of said soft tube (11) toward said proximal end thereof, a second bubble-containing surface layer (13b) extended in a predetermined width from said distal end of said soft tube (11) toward said proximal end thereof and opposed to said first bubble-containing surface layer (13a), a first colorless and transparent part (15a) positioned between said first (13a) and second (13b) bubble-containing surface layers, extended in a predetermined length from said distal end of said soft tube (11) toward said proximal end thereof, and allowing an inside of said lumen to be visually recognized by microscopic observation, and a second colorless and transparent part (15b) provided opposite to said first colorless and transparent part (15a); and
each of said first (13a) and second (13b) bubble-containing surface layers has a lot of bubbles set long in an axial direction of said soft tube (11);
**characterized in that**
a thickness of each of said first (13a) and second (13b) bubble-containing surface layers is set to 1/5 to 1/3 of a thickness of said soft tube (11); a width of each of said first (13a) and second (13b) bubble-containing surface layers is set to 5/100 to 20/100 of an outer circumferential length of said soft tube (11); and a width of each of said first (15a) and second (15b) colorless and transparent parts is set to not less than 30/100 of said outer circumferential length of said soft tube (11).

2. An embryo transfer tool (1) according to claim 1, wherein a bubble volume content of an outer portion of each of said first (13a) and second (13b) bubble-containing surface layers is set higher than that of an inner portion thereof.

3. An embryo transfer tool (1) according to claim 1 or 2, wherein said thickness of each of said first (15a) and second (15b) bubble-containing surface layers is set to 1/5 to 1/4 of said thickness of said soft tube (11).

4. An embryo transfer tool (1) according to any one of claims 1 through 3, wherein said width of each of said first (15a) and second (15b) bubble-containing surface layers is set to 7/100 to 15/100 of said outer circumferential length of said soft tube.

5. An embryo transfer tool (1) according to any one of claims 1 through 4, wherein a length (L)of a bubble (14) in said axial direction of said soft tube (11) is set to not less than twice as long as a width (W) of said bubble (14).

6. An embryo transfer tool (1) according to any one of claims 1 through 5, wherein a bubble volume content of said bubble-containing surface layer is set to 3 to 15 percent by volume.

7. An embryo transfer tool (1) according to any one of claims 1 through 6, which has a plurality of markers (35, 23) disposed at regular intervals at said proximal end portion, of said soft tube, which is located distally from said hub (12, 22).

8. An embryo transfer device comprising an embryo transfer tool (1) according to any one of claims 1 through 7 and a sheath (2) having a flexible tube harder (21) than said soft tube (11) and a sheath hub (22) provided at a proximal end of said flexible tube (12),
wherein said sheath (2) accommodates said soft tube (11) with a distal part of said soft tube (11) of said embryo transfer tool (1) projecting from a distal end of said sheath (2).

9. An embryo transfer device according to claim 8, wherein said soft tube (11) has said first (13a) and second (13b) bubble-containing surface layers over an entire length of a part of said sheath (2) projected from said distal end thereof.

10. An embryo transfer device according to claim 8 or 9, wherein said sheath (2) has a plurality of markers (35, 23) formed at regular intervals at a distal part thereof.

## Patentansprüche

1. Embryotransfer-Instrument (1), umfassend einen weichen Schlauch (11), der ein Lumen aufweist, das durch diesen von einem distalen Ende davon zu einem proximalen Ende davon hindurchgeht, und eine Nabe (12, 22), die an einem proximalen Endabschnitt des weichen Schlauchs (11) vorgesehen ist,
wobei der weiche Schlauch (11) eine erste Blasen enthaltende Oberflächenschicht (13a) aufweist, die sich in einer vorbestimmten Breite und in einer vorbestimmten Länge von dem distalen Ende des weichen Schlauchs (11) zu dem proximalen Ende davon erstreckt, eine zweite Blasen enthaltende Oberflächenschicht (13b), die sich in einer vorbestimmten Breite von dem distalen Ende des weichen Schlauchs (11) in Richtung des proximalen Endes davon erstreckt und der ersten Blasen enthaltenden Oberflächenschicht (13a) gegenüberliegt, einen ersten farblosen und transparenten Teil (15a), der zwischen der ersten (13a) und der zweiten (13b) Blasen enthaltenden Oberflächenschicht angeordnet ist, der sich in einer vorbestimmten Länge von dem distalen Ende des weichen Schlauchs (11) in Richtung des proximalen Endes davon erstreckt und es ermöglicht, eine Innenseite des Lumens durch mikroskopische Beobachtung visuell zu erkennen, und einen zweiten farblosen und transparenten Teil (15b), der gegenüber dem ersten farblosen und transparenten Teil (15a) vorgesehen ist; und
jede der ersten (13a) und zweiten (13b) Blasen enthaltenden Oberflächenschichten eine Menge von Blasen aufweist, die in axialer Richtung des weichen Schlauches (11) länglich eingelassen sind;
**dadurch gekennzeichnet, dass**
eine Dicke von jeder der ersten (13a) und zweiten (13b) Blasen enthaltenden Oberflächenschichten auf 1/5 bis 1/3 einer Dicke des weichen Schlauchs (11) festgesetzt ist; eine Breite von jeder der ersten (13a) und zweiten (13b) Blasen enthaltenden Oberflächenschichten auf 5/100 bis 20/100 einer äußeren Umfangslänge des weichen Schlauchs (11) festgesetzt ist; und eine Breite von jedem der ersten (15a) und zweiten (15b) farblosen und transparenten Teile auf nicht weniger als 30/100 der äußeren Umfangslänge des weichen Schlauchs (11) festgesetzt ist.

2. Embryotransfer-Instrument (1) nach Anspruch 1, wobei der Blasenvolumengehalt eines äußeren Abschnitts von jeder der ersten (13a) und der zweiten (13b) Blasen enthaltenden Oberflächenschicht höher festgesetzt ist als der eines inneren Abschnitts davon.

3. Embryotransfer-Instrument (1) nach Anspruch 1 oder 2, wobei die Dicke von jeder der ersten (15a) und der zweiten (15b) Blasen enthaltenden Oberflächenschicht auf 1/5 bis 1/4 der Dicke des weichen Schlauchs (11) festgesetzt ist.

4. Embryotransfer-Instrument (1) nach einem der Ansprüche 1 bis 3, wobei die Breite von jeder der ersten (15a) und der zweiten (15b) Blasen enthaltenden Oberflächenschicht auf 7/100 bis 15/100 der äußeren Umfangslänge des weichen Schlauchs festgesetzt ist.

5. Embryotransfer-Instrument (1) nach einem der Ansprüche 1 bis 4, wobei eine Länge (L) einer Blase (14) in der axialen Richtung des weichen Schlauchs (11) auf nicht weniger als die doppelte Länge einer Breite (W) der Blase (14) festgesetzt ist.

6. Embryotransfer-Instrument (1) nach einem der Ansprüche 1 bis 5, wobei ein Blasenvolumengehalt der Blasen enthaltenden Oberflächenschicht auf 3 bis 15 Volumenprozent festgesetzt ist.

7. Embryotransfer-Instrument (1) nach einem der Ansprüche 1 bis 6, das eine Vielzahl von Markierungen (35, 23) aufweist, die in regelmäßigen Abständen an dem proximalen Endabschnitt des weichen Schlauchs angeordnet sind, der distal von der Nabe (12, 22) angeordnet ist.

8. Vorrichtung zum Embryotransfer, umfassend ein Embryotransfer-Instrument (1) nach einem der Ansprüche 1 bis 7 und eine Hülle (2), die einen flexiblen Schlauch (21) aufweist, der härter als der weiche Schlauch (11) ist, und eine Hüllennabe (22), die an einem proximalen Ende des flexiblen Schlauchs (12) vorgesehen ist,
wobei die Hülle (2) den weichen Schlauch (11) aufnimmt, wobei ein distaler Teil des weichen Schlauchs (11) des Embryotransfer-Instrumentes (1) von einem distalen Ende der Hülse (2) hervorsteht.

9. Vorrichtung für den Embryotransfer nach Anspruch 8, wobei der weiche Schlauch (11) die erste (13a) und die zweite (13b) Blasen enthaltende Oberflächenschicht über die gesamte Länge eines Teils der Hülle (2) aufweist, der aus dem distalen Ende der Hülle hervorsteht.

10. Vorrichtung für den Embryotransfer nach Anspruch 8 oder 9, wobei die Hülle (2) eine Vielzahl von Markierungen (35, 23) aufweist, die in regelmäßigen Abständen an einem distalen Teil davon ausgebildet sind.

## Revendications

1. Instrument de transfert embryonnaire (1) comprenant un tube souple (11) ayant une lumière qui le traverse d'une extrémité distale de celui-ci à une extrémité proximale de celui-ci et un moyeu (12, 22) prévu au niveau d'une portion d'extrémité proximale dudit tube souple (11),
dans lequel ledit tube souple (11) a une première couche de surface contenant des bulles (13a) s'étendant sur une largeur prédéterminée et sur une longueur prédéterminée depuis ladite extrémité distale dudit tube souple (11) vers ladite extrémité proximale de celui-ci, une seconde couche de surface contenant des bulles (13b) s'étendant sur une largeur prédéterminée depuis ladite extrémité distale dudit tube souple (11) vers ladite extrémité proximale de celui-ci et opposée à ladite première couche de surface contenant des bulles (13a), une première partie incolore et transparente (15a) positionnée entre lesdites première (13a) et seconde (13b) couches de surface contenant des bulles, s'étendant sur une longueur prédéterminée depuis ladite extrémité distale dudit tube souple (11) vers ladite extrémité proximale de celui-ci, et permettant de reconnaître visuellement un intérieur de ladite lumière par observation microscopique, et une seconde partie incolore et transparente (15b) prévue à l'opposé de ladite première partie incolore et transparente (15a) ; et
chacune desdites première (13a) et seconde (13b) couches de surface contenant des bulles a un grand nombre de bulles fixées en longueur dans une direction axiale dudit tube souple (11) ;
**caractérisé en ce que**
une épaisseur de chacune desdites première (13a) et seconde (13b) couches de surface contenant des bulles est fixée à 1/5 à 1/3 d'une épaisseur dudit tube souple (11) ; une largeur de chacune desdites première (13a) et seconde (13b) couches de surface contenant des bulles est fixée à 5/100 à 20/100 d'une longueur circonférentielle extérieure dudit tube souple (11) ; et une largeur de chacune desdites première (15a) et seconde (15b) parties incolores et transparentes est fixée à pas moins de 30/100 de ladite longueur circonférentielle extérieure dudit tube souple (11).

2. Instrument de transfert embryonnaire (1) selon la revendication 1, dans lequel une teneur en volume de bulles d'une portion extérieure de chacune desdites première (13a) et seconde (13b) couches de surface contenant des bulles est fixée plus haut que celle d'une portion intérieure de celles-ci.

3. Instrument de transfert embryonnaire (1) selon la revendication 1 ou 2, dans lequel ladite épaisseur de chacune desdites première (15a) et seconde (15b) couches de surface contenant des bulles est fixée à 1/5 à 1/4 de ladite épaisseur dudit tube souple (11).

4. Instrument de transfert embryonnaire (1) selon l'une quelconque des revendications 1 à 3, dans lequel ladite largeur de chacune desdites première (15a) et seconde (15b) couches de surface contenant des bulles est fixée à 7/100 à 15/100 de ladite longueur circonférentielle extérieure dudit tube souple.

5. Instrument de transfert embryonnaire (1) selon l'une quelconque des revendications 1 à 4, dans lequel une longueur (L) d'une bulle (14) dans ladite direction axiale dudit tube souple (11) est fixée à pas moins du double d'une largeur (W) de ladite bulle (14).

6. Instrument de transfert embryonnaire (1) selon l'une quelconque des revendications 1 à 5, dans lequel une teneur en volume de bulles de ladite couche de surface contenant des bulles est fixée à 3 à 15 pour cent en volume.

7. Instrument de transfert embryonnaire (1) selon l'une quelconque des revendications 1 à 6, qui a une pluralité de marqueurs (35, 23) disposés à intervalles réguliers au niveau de ladite portion d'extrémité proximale, dudit tube souple, qui est situé de manière distale par rapport audit moyeu (12, 22).

8. Dispositif de transfert embryonnaire comprenant un instrument de transfert embryonnaire (1) selon l'une quelconque des revendications 1 à 7 et une gaine (2) ayant un tube flexible plus dur (21) que ledit tube souple (11) et un moyeu de gaine (22) prévu au niveau d'une extrémité proximale dudit tube flexible (12),
dans lequel ladite gaine (2) reçoit ledit tube souple (11), avec une partie distale dudit tube souple (11) dudit instrument de transfert embryonnaire (1) faisant saillie à partir d'une extrémité distale de ladite gaine (2).

9. Dispositif de transfert embryonnaire selon la revendication 8, dans lequel ledit tube souple (11) a lesdites première (13a) et seconde (13b) couches de surface contenant des bulles sur toute une longueur d'une partie de ladite gaine (2) en saillie à partir de ladite extrémité distale de celui-ci.

10. Dispositif de transfert embryonnaire selon la revendication 8 ou 9, dans lequel ladite gaine (2) a une pluralité de marqueurs (35, 23) formés à intervalles réguliers au niveau d'une partie distale de celle-ci.
